# EUROPEAN PATENT SPECIFICATION

(11) **EP 1 957 165 B1**
(45) Date of publication and mention of the grant of the patent: **09.02.2011**
(21) Application number: 06812966.7
(22) Date of filing: 02.11.2006
(51) Int. Cl.: A61N 5/10, G01T 1/16, G01T 1/161

(54) **A RADIATION MONITORING DEVICE PROVIDED WITH MEANS TO MEASURE AN ADMINISTRATED DOSE IN A TARGET AREA**
STRAHLENÜBERWACHUNGSVORRICHTUNG MIT MITTELN ZUM MESSEN EINER VERABREICHTEN DOSIS IN EINEM ZIELGEBIET
APPAREIL DE CONTRÔLE DE RADIATIONS OFFRANT DES MOYENS POUR MESURER UNE DOSE ADMINISTRÉE DANS UNE RÉGION CIBLE

(30) Priority: 28.11.2005 SE 0502594
(43) Date of publication of application: 20.08.2008
(73) Proprietor: Micropos Medical AB, 412 92 Göteborg (SE)
(72) Inventor: ROSENGREN, Bengt, S-436 51 Hovås (SE); LENNERNÄS, Bo, S-451 44 Uddevalla (SE); IUSTIN, Roman, S-431 67 Mölndal (SE); GUSTAFSSON, Tomas, S-431 67 Mölndal (SE)
(74) Representative: Hedman, Anders
(86) International application number: PCT/SE2006/001242
(87) International publication number: WO 2007/061351

(56) References cited:
- EP-A2- 1 050 321
- WO-A-01/66156
- WO-A-03/047694
- WO-A-2004/080522
- WO-A1-00/40299
- WO-A1-97/17595
- WO-A1-02/079803
- WO-A1-02/100485
- WO-A2-03/047694
- FR-A- 2 706 628
- US-A- 4 959 547
- US-A1- 2005 012 043
- US-B1- 6 241 670

## Description

### Technical field

The present invention relates to a radiation monitoring device for locating a target area and measure an administered dose, provided from a radiation source, in the target area.

### Background to the invention

Today the administered dose is normally only measured on the outside of the human body during radiotherapy. Due to the lossy nature of the human body the radiation starts to disperse.

There already exists a directive in Denmark with the object to ensure that it should be possible to measure the dose if an irradiation source is used. This regulation is projected to be adopted by other countries, such as France, and it could also become a standard regulation for radiation treatment.

In an article with the title "An implantable radiation dosimeter for use in external beam radiation therapy", by Charles W. Scarantino et al., published in American Association of Physicists in Medicine, September 2004, pages 2658 - 2670, a method for measuring administered dose in a target area is disclosed. An implantable radiation dosimeter is introduced into a living body and telemetrically read using inductive power on a daily basis, i.e. before and after a radiotherapy session. A disadvantage with the disclosed system is that it is not possible to monitor the administered dose during each radiotherapy session and therefore can not be used to control the amount of radiation the patient is subjected to during radiotherapy. Another disadvantage is that the dosimeter can not be removed from the patient after completion of the radiotherapy without a surgical operation.

In WO 01/66156 a radiation monitoring device is disclosed having internal elements for tracking a position of the device, a catheter, a marker and a movable unit provided with a dose detector. The lumen of the catheter provides a mechanical guide for the movable unit when placed within the catheter.

In US 2005/012043 a detector unit is disclosed having a dose detector and a radio-opaque marker suitable for tracking the position of the device. The unit is disposed within a catheter and the lumen of the catheter acts as a mechanical guide for the detector unit, which is rotatable and movable within the catheter.

### Summary of the invention

An object with the present invention is to provide a radiation monitoring device that is provided with means to determine a position of a target area and that measures the administrated dose of radiation during a radiotherapy session in the target area, and therefore may achieve a more accurate level of radiation in the target area of a patient compared to prior art.

The object is achieved by a device which is fixated relative a target area within a living body. The radiation monitor device comprises in addition to a dose measuring device an internal element used to track variations of a position of the device in relation to a radiation source arranged outside the target area. The purpose of the dose measuring device is to detect an administered dose in the target area from the radiation source.

An advantage with the present invention is that the actual administered dose inside a target area could be more or less continuously detected, depending on the sampling rate of the measuring equipment.

Another advantage with the present invention is that a cumulative administered dose can be calculated for a patient which will increase the possibility to monitor the administered dose during a series of radiation treatments.

Still another advantage is that the present invention is cheap and easy to implement. Furthermore, the quality of the treatment may be documented and also improved.

In a further embodiment the radiation monitoring device is implemented as an implant, wherein the administered dose in a target area inside a living body may be monitored.

In an alternative embodiment, the radiation monitoring device is implemented as a skin adhering item, such as a plaster, wherein the administered dose in a target area situated close to the skin of a patient may be monitored.

Further objects and advantages will be apparent for a skilled person from the detailed description and the drawings.

### Brief description of the drawings

Fig. 1 shows a schematic view of a first embodiment of a radiation monitoring device in a living body.
Fig. 2 shows a block diagram of a first embodiment of a dose measuring unit in the implant of figure 1.
Fig. 3 shows a cross-sectional view of a second embodiment of a radiation monitoring device in a living body.
Fig. 4 shows a block diagram of a second embodiment of a dose measuring unit in the radiation monitoring device of figure 3.
Fig. 5 shows a cross-sectional view of a third embodiment of a radiation monitoring device.
Fig. 6 shows a cross-sectional view of a fourth embodiment of a radiation monitoring device.
Fig. 7 shows a system implementing a monitoring device according to the invention.
Fig. 8 shows a plot illustrating the near field effect of electromagnetic signals.
   Fig. 9 shows a system with an alternative implementation of a monitoring device according to the invention.

### Detailed description of the preferred embodiments

Radiotherapy normally contains several radiation sessions, for instance between 8 and 35, spread out during a predetermined time period for treating cancer, e.g. prostate cancer, and each radiation session comprises normally several fractions during which the patient is subjected to radiation. Prior art suggest how the administered dose can be measured between each radiation session, and the present invention also provide a means to measure the administrated dose between each fraction or even during a fraction. This will improve the possibility to more accurately monitor the administrated dose and furthermore, limit the patient's exposure to radiation, since the predetermined dose, e.g. 70-100 Gray, can be used to control the amount of radiation in a present or subsequent fraction.

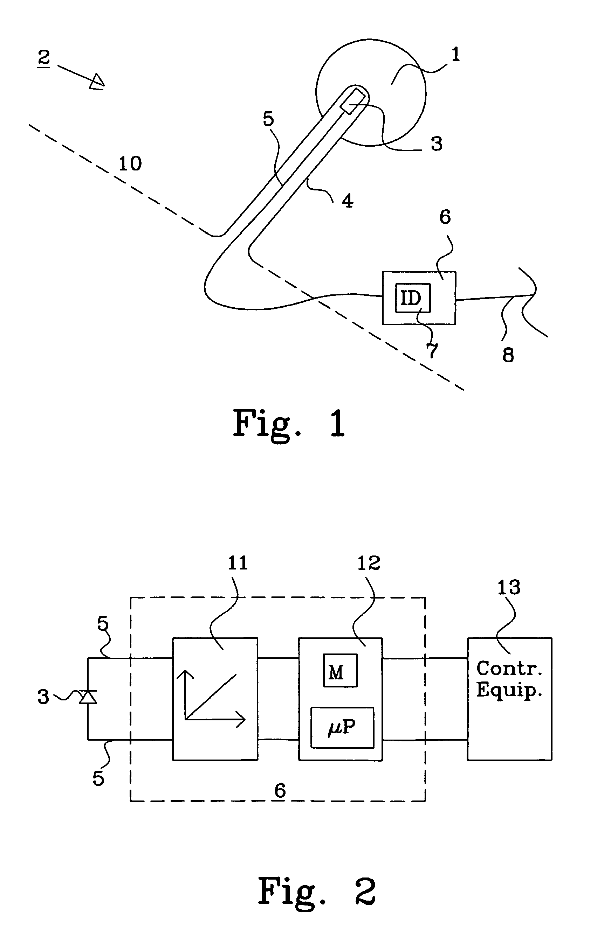

Figure 1 shows a schematic view of a first embodiment of a radiation monitoring device 2, in this embodiment an implant, in a living body 10 of a patient. A bio-compatible cover 4, such as a catheter, is provided either through the tissue or through a natural opening in the body. The procedure of inserting the catheter is common knowledge of a skilled person in the art, e.g. Seldinger technique.

The catheter is inserted into, or near by, a target area 1, e.g. a cancer tumor. A dose sensor 3 is inserted into the catheter 4 and is connected through wires 5 to an externally arranged dose conversion unit 6. The dose conversion unit 6 is preferably provided with an electronic ID, or a written ID-label 7 (printed or hand written), and is described in more detail in figure 2.

The dose conversion unit 6 converts the signal from the dose sensor to an amount of administrated dose generated by the radiation source and is preferably fastened to the outside of the patient using an adhering material, such as a plaster. The conversion unit is in turn connected to a control equipment (not shown) through a connection 8 for analyzing the measured dose by the measuring unit 6.

When the treatment of the patient is completed, the catheter including the dose sensor 3 is thereafter removed from the patient and no part of the implant 2 is left in the living body in this embodiment after completion. The catheter is constructed in such a way that it is held in place during the radiation treatment, but may easily be removed when it is completed. An example of fastening mechanisms is a multiple of tine elements such as disclosed in the published US application US 2006/0129218, assigned to Medtronic, Inc. Such fastening mechanisms are known to a person skilled in the art and are therefore not described in more detail.

Figure 2 shows a block diagram of a first embodiment of a dose conversion unit 6 in the implant of figure 1. The dose sensor 3, such as at least one diode, is arranged inside the catheter (not shown) and is connected through wires 5 to an integrator 11 that continuously measures the administrated dose. The integrator 11 could for instance be an op-amp (operational amplifier) with a capacitor in its feedback loop, and a certain level of irradiation results in a certain level of DC (Direct Current) voltage. If a diode is used, it normally needs to be pre-radiated to create a linear response from the diode.

The integrator 11 is connected to a processing unit 12 which comprises a microprocessor µP, and an internal memory M, but an external memory may naturally be used instead. The processing unit 12 receives information from the integrator 11 regarding the administered dose during a treatment and may forward this information to a control equipment 13. The control equipment may use this information to in turn control the amount of radiation irradiated on the target area by a radiation equipment (not shown). A cumulative value corresponding to the total measured administered dose is also calculated and stored in the memory M. The calculations are preferably performed within the microprocessor µP.

Each dose sensor is normally tested and characterized before use and calibration information is obtained to provide a simple normalized interface between the dose conversion unit 6 and the external control and radiation equipment. This information may be stored in the memory M or stored externally, but it needs to be accessible to provide a means to interpret the measured dose and translate it into an absolute value of administered radiation in the target area 1.

An electronic ID tag may also be stored in the memory M together with information regarding the scheduled radiation treatment for the patient. This information may be used to further ensure that the right amount of radiation is given to the correct patient by comparing the entered ID on the radiation equipment and/or control equipment and comparing it with the stored ID in the memory of the dose conversion unit 6.

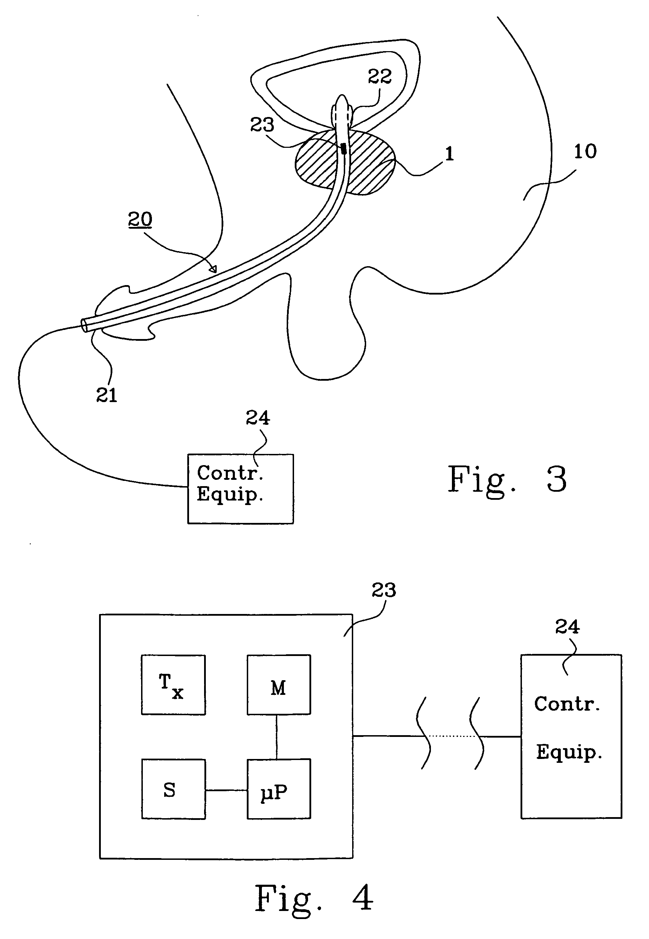

Figure 3 shows a cross-sectional view of a second embodiment of a radiation monitoring device 20, in this embodiment an implant, in a living body 10. The catheter 21 is in this example introduced through a natural opening and will assist in determining the administered dose when treating for instance prostate cancer. An inflatable balloon 22, which is part of the catheter 21, helps to securely position the implant relative a target area 1. A dose conversion unit 23 is arranged within the catheter 21 within the target area 1. The catheter is removed after each treatment occasion by deflating the balloon and removing the implant 20. If the catheter is provided with a through going opening, such as a lumen, the bladder may be emptied even though the catheter is maintained in position.

Figure 4 shows a block diagram of the second embodiment of a dose conversion unit 23 in the implant of figure 3, and comprises in this example a transmitter Tₓ used for determining the position of the target area 1 (and thus the dose conversion unit 23), as is disclosed in the international publication WO 2005/104976, which is assigned to the same applicant.

The dose conversion unit 23 further comprises a sensor S, a microprocessor µP and a memory M. The sensor includes in this embodiment at least one MOSFET preferably, but not necessarily, connected to an op-amp. The MOSFET does not have to be pre-radiated and an integrator is not needed since the MOSFET measures the absolute dose after completed irradiation of the target area 1.

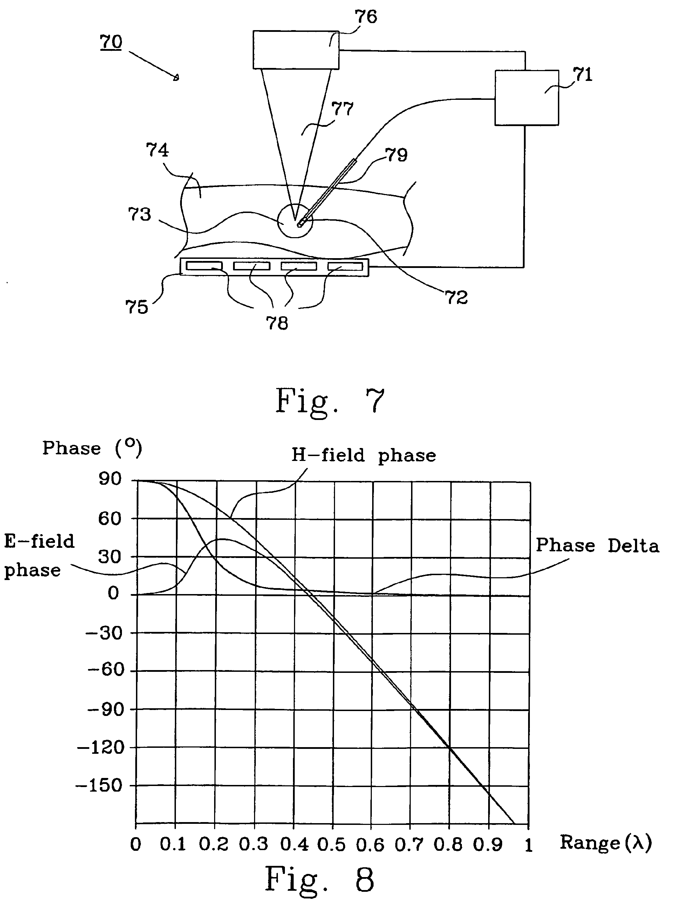

The dose conversion unit 23 is further connected to a control equipment 24 which is provided with antennas (not shown) to determine the position of the transmitter Tₓ. Signals are generated in the control unit 24, transmitted to the transmitter Tₓ which emits signals that are received by the antennas to determine the position of the target area 1. This system has been disclosed in the above mentioned WO 2005/104976 and is briefly described together with the present invention in connection with figure 7.

An electromagnetic signal having a frequency within the range of 5-350 MHz is generated in a control unit 71 and is thereafter transmitted from at least one transmitter 72, included in a radiation monitor device 79 according to the invention, arranged in relation to a target area 73 inside a living body 74. The electromagnetic signal is adapted to propagate with a wavelength in the body 74 and, in a first example, a phase difference of the electromagnetic signal is detected in at least three positions 78, preferably four or more positions, by a receiver 75 arranged outside the body 74. The wavelength is selected so that a distance from the transmitter 11 to each of said at least three positions 78 is within the same integer number of wavelengths of the electromagnetic signal. The distance between each transmitter 72 and the positions of the receiver 75 is preferably selected so that they operate in a near field region. In this example a radiation source 76 is arranged outside the body 74 and preferably connected to the control unit 71. The radiation beam 77 of the radiation source 76 is concentrated to a treatment area. Furthermore, the transmitter 72 is in this embodiment arranged within the target area 73, which in this example is identical to the treatment area of the patient.

The behaviour of an electromagnetic signal in the near field region is known for a skilled person and is described in a publication with the title "Near field Phase Behavior", by Hans Gregory Schantz, IEEE APS Conference July 2005. In this publication the author presents a reprint of a plot published in "Electric waves", by Heinrich Hertz, London, Macmillian & Co. 1893, page 152 and a plot, shown in figure 8, was published by Q-track in 2004.

The plot describes the phase behaviour of the magnetic field (H-field) and the electrostatic filed (E-field) below one wavelength of an electromagnetic signal. In this near field region of an antenna, the magnetic field and electrostatic field phases radically diverge, and in a far field region, many wavelengths away from a transmit antenna, the magnetic and electrostatic field move with perfect synchronized phase. Figure 8 illustrates the effect of the near field region, and the phase delta between the magnetic field and electrostatic field at zero λ is 90 degrees, which decreases to a phase difference of 0 degrees at one λ.

The separation of the magnetic field and the electrostatic field in the near field region opens up a number of possibilities to construct improved measurement systems. The shape of the wave front of the electromagnetic signal may be used to determine the distance between the transmitter and the receiver. It is also advantageous to increase the sensitivity of the measurement system by introducing electrostatically shielded antennas, which is possible since the magnetic field and electrostatic field are separated in the near field region, whereby the magnetic field is used to determine the variations of the position of the transmitter.

A more detailed description of the detector system may be found in the international patent application with the publication number WO 2005/104976, which is hereby incorporated by reference.

In a second example, amplitude difference of the electromagnetic signal is detected instead of the phase difference as described above in connection with figure 7. At least one transmitter 72 arranged in relation to a target area 73 inside a body 74 transmits a signal having a frequency within the range of 1 kHz-350 MHz and an amplitude difference from the transmitted signal is detected by a receiver 75 at three, or more, positions 78 to track variations in position of the transmitter 72. The amplitude of the magnetic field is preferably measured when operating in near field, for instance by measuring absolute value or mean value of the magnetic field.

It is of course possible to combine the above described examples and use both phase and amplitude difference to determine variations of the position of the transmitter 72 in relation to the positions 78 of the receiver 75.

The receiver 75, comprising a multiple of antennas in at least three separate positions 78 (in this example four positions), is positioned on the outside of the body and is also connected to the control unit 71, and a very accurate tracking of the transmitter 72, and thus the target area 73, may be performed.

In an alternative embodiment signals are transmitted from at least one externally arranged transmitter, and the electromagnetic signal is received by a receiver inside the radiation monitoring device. The receiver comprises three antennas in at least three separate positions. The separate position of the antennas of the receiver may be accomplished by moving the antenna in a predetermined pattern, or by providing three or more antennas.

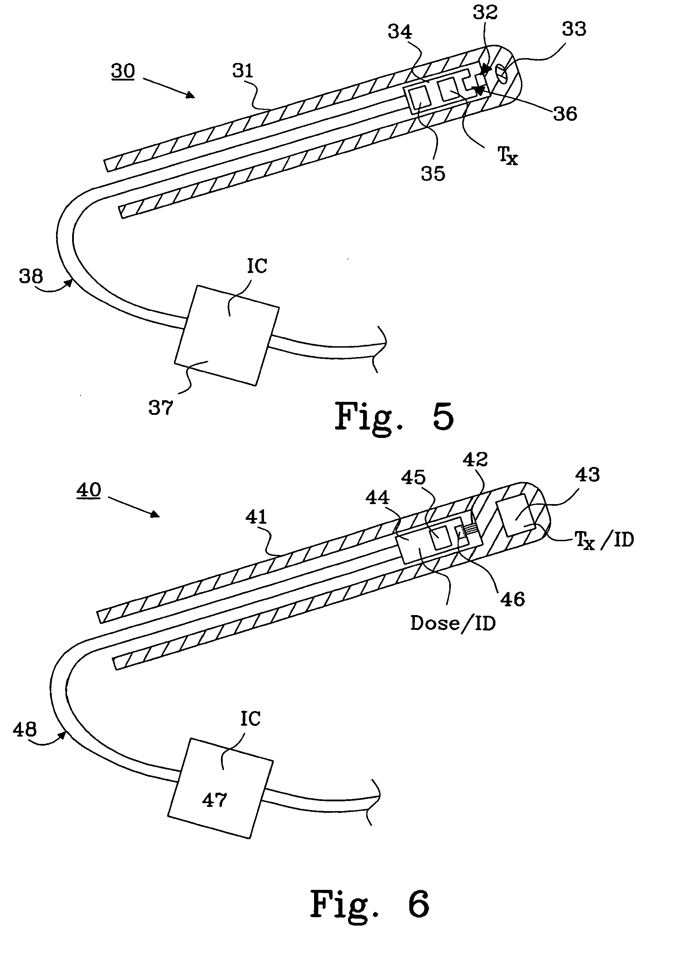

Fig. 5 shows a cross-sectional view of a third embodiment of a radiation monitoring device 30, in this embodiment an implant, comprising a catheter 31 provided with a mechanical guide 32 and a marker 33. The marker 33 is visible when the implant is subjected to x-ray radiation. The implant further comprises a combined dose and positioning unit 34 having a transmitter Tₓ used to determine the position of the target area in a patient, and a dose sensor 35 used to detect the amount of administered dose in the target area.

The combined dose and positioning unit 34 is provided with a guide element 36 that is arranged to be able to mate with the mechanical guide 32 and will ensure that the unit 34 is held in the correct position during radiation treatment. The combined dose and positioning unit 34 is connected to an externally arranged integrated circuit 37 through wires 38, and may also be withdrawn from the catheter 31 between the treatment occasions. The integrated circuit 37 includes the functionality needed to perform the dose conversion as previously described in connection with figures 1-4.

In an alternative embodiment, the transmitter Tₓ is omitted and the marker 33 is used as a positioning device during radiation, since the marker 33 is visible when the monitoring device is subject to x-ray radiation. A positioning system using implanted markers has been proposed by Initia Medical Technologies.

Fig. 6 shows a cross-sectional view of a fourth embodiment of a radiation monitoring device 40, in this embodiment an implant, and comprises a catheter 41 provided with an electrical guide 42 and an electrical marker 43. The marker 43 comprises a transmitter Tₓ used to determine the position of the target area in a patient and an identification ID of the patient. The implant further comprises a combined dose and identification unit 44 having a dose sensor 45 used to detect the amount of administered dose in the target area and a dose identification Dose/ID.

The combined dose and identification unit 44 is provided with a connector 46 that is arranged to be able to connect to the electrical guide 42 and will ensure that the correct unit 44 is connected by comparing the dose identification Dose/ID and the ID of the patient in the electrical marker 43, and verify that it is held in the correct position during radiation treatment since the transmitter Tₓ is powered through the combined dose and identification unit 44. The combined dose and identification unit 44 is connected to an externally arranged integrated circuit 47 through wires 48, and may also be withdrawn from the catheter 41 between the treatment occasions. The integrated circuit 47 includes the functionality needed to perform the dose conversion as previously described in connection with figures 1-4.

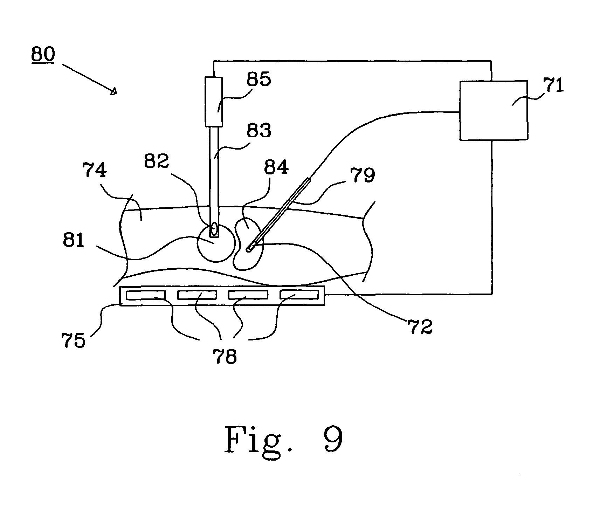

Figure 9 shows a system with an alternative implementation of a monitoring device according to the invention. The hardware of the systems could be identical to the system described in connection with figure 7, but a system adapted for brachytherapy is shown in figure 9. The radiation source, in the form of seeds 82, is configured to be introduced inside a treatment area 81 using catheters 83, or needles, instead of exposing the treatment area 81 to x-ray radiation using an externally arranged radiation source 76 as shown in figure 7. The radiation monitoring device 79 is in this implementation arranged in a target area 84, which is a vital organ adjacently arranged to the treatment area 81. The vital organ in the target area 84 should be exposed to a minimal radiation dose, and by monitoring the administered dose in the target area 84 when treating the treatment area 81 the amount of radiation emitted from the radiation source 82 may be controlled. In other words, the vital organ is arranged in a non-treatment area. A positioning device 85, which controls the actual position of the tip of the catheter 83, and thus the radiation source 82, is preferably connected to the control unit 71.

Other types of sensors may naturally be added in the radiation monitoring device to further monitor other data from the target area, such as pressure, pH, temperature, oxygen saturation, drug concentration, etc. which implementations are obvious for a skilled person in the art and will therefore not be described in more detail.

In the above described embodiments, electromagnetic signals have been described to determine the position of the radiation monitoring device, but the scope of the invention should not be limited to this. A magnetically excitable internal element that communicates with at least one externally arranged detector is also possible.

The above described embodiments of the invention exemplifies the inventive concept any combination of the described embodiment is conceivable. Thus, the scope of the invention should not be limited to the specific embodiments.

## Claims

1. A radiation monitoring device (30; 40;79) fixable relative to a target area (73) within a living body (74), wherein said device comprises at least one internal element (Tₓ; 72) for tracking variations of a position of the device relative to a radiation source (76) arranged outside said target area (73) during radiotherapy, said device comprising:
- a catheter (31; 41) provided with a guide (32; 42) and a marker (33; 43), and
- a withdrawable unit (34; 44), provided with a guide element (36; 46) and a dose measuring device configured to detect an administrated dose in the target area from the radiation source when the withdrawable unit (34; 44) is positioned within the catheter (31; 41) and the guide (32; 42) mate with the guide element (36; 46) in the catheter (31; 41), **characterized in that** said radiation monitoring device further comprises identification means (43) to identify the living body to ensure that the administrated dose from the radiation source is radiated into a correct living body.

2. The radiation monitoring device according to claim 1, wherein said dose measuring device comprises at least a dose sensor (35; 45) arranged in the vicinity of the internal element (Tₓ; 72), a conversion unit (37; 47) to convert the signal from the dose sensor (35; 45) to an amount of dose delivered to the target area, said conversion unit (37; 47) is located outside of the living body.

3. The radiation monitoring device according to claim 1 or 2, wherein said identification means comprises an ID-tag stored in a memory connected to said conversion unit (37; 47).

4. The radiation monitoring device according to claim 3, wherein said device further comprises means (37; 47) to calculate a value equal to the accumulated administered dose and save the calculated value in the memory.

5. The radiation monitoring device according to any of claims 1-4, wherein said withdrawable unit (44) is a combined dose and identification unit.

6. The radiation monitoring device according to any of claims 1-4, wherein said guide is a mechanical guide (32) configured to mate with said guide element (36) when said withdrawable unit (34) is inserted into said catheter (31) to ensure that the withdrawable unit (34) is held in a correct position during radiation treatment.

7. The radiation monitoring device according to claim 6, wherein said withdrawable unit (34) is a combined dose and positioning unit.

8. The radiation monitoring device according to any of claims 1-5, wherein said guide element is a connector (46) and said guide is an electrical guide (42) configured to connect to the connector (46) when said withdrawable unit (44) is inserted into said catheter (41) to ensure that the withdrawable unit (44) is held in a correct position during radiation treatment.

9. The radiation monitoring device according to claim 8, wherein the marker is an electrical marker (43) configured to be connected to the withdrawable unit (44) through said connector (42) and electrical guide (46).

10. The radiation monitoring device according to any of claims 1-9, wherein each internal element is an internal antenna element arranged to communicate using electromagnetic signals with at least one externally arranged antenna element (75) outside the living body for tracking the position of the device relative to said at least one externally arranged antenna element (75).

11. The radiation monitoring device according to claim 10, wherein each internal antenna element is configured to communicate using an electromagnetic signal that is adapted to propagate with a wavelength in said living body so that a phase difference of said electromagnetic signal is detectable for tracking variations of the position of each internal antenna element relative to each externally arranged antenna element.

12. The device according to any of claims 10-11, wherein each internal antenna element is configured to communicate using an electromagnetic signal that is adapted to propagate with a wavelength in said body so that an amplitude difference of said electromagnetic signal is detectable for tracking variations of the position of each internal antenna element relative to each externally arranged antenna element.

13. The radiation monitoring device according to any of claims 10-12, wherein said at least one internal antenna element is a transmitter (Tₓ; 72) arranged to emit the electromagnetic signals, which is detectable in at least three positions (78) by the at least one externally arranged antenna element (75).

14. The radiation monitoring device according to any of claims 1-11, wherein said radiation monitoring device is an implant (30; 40; 79) and at least a part of the implant is positioned in the target area, and said implant is configured to be connected to an externally arranged control unit (71).

15. A system comprising the radiation monitoring device (30; 40; 79) according to any of claims 1-14 and said radiation source (76) configured to be arranged outside said living body (74), wherein said radiation monitoring device (79) is configured to be introduced into a treatment area (73) inside said living body.

## Patentansprüche

1. Strahlenüberwachungsvorrichtung (30; 40; 79), die relativ zu einem Zielbereich (73) in einem lebenden Körper (74) fixiert werden kann, wobei die Vorrichtung mindestens ein internes Element (Tₓ; 72) umfasst, um während der Strahlentherapie Positionsänderungen der Vorrichtung relativ zu einer außerhalb des Zielbereichs (73) angeordneten Strahlenquelle (76) zu verfolgen, wobei die Vorrichtung umfasst:
- einen Katheter (31; 41), der mit einer Führung (32; 42) und einer Markierung (33; 43) ausgestattet ist, und
- eine entnahmefähige Einheit (34; 44), die mit einem Führungselement (36; 46) und einer Dosismessvorrichtung ausgestattet ist, die konfiguriert ist, um eine von der Strahlenquelle verabreichte Dosis in dem Zielbereich zu detektieren, wenn die entnahmefähige Einheit (34; 44) innerhalb des Katheters (31; 41) positioniert ist und die Führung (32; 42) sich mit dem Führungselement (36; 46) in dem Katheter (31; 41) vereinigt, **dadurch gekennzeichnet dass** die Strahlenüberwachungsvorrichtung ferner Identifizierungsmittel (43) umfasst, um den lebenden Körper zu identifizieren, damit gewährleistet ist, dass die von der Strahlenquelle verabreichte Dosis in einen korrekten lebenden Körper eingestrahlt wird.

2. Strahlenüberwachungsvorrichtung nach Anspruch 1, wobei die Dosismessvorrichtung mindestens einen Dosissensor (35; 45), der in der Nähe des internen Elements (Tₓ; 72) angeordnet ist, und eine Umwandlungseinheit (37; 47) umfasst, um das Signal von dem Dosissensor (35; 45) in eine in den Zielbereich abgegebene Dosismenge umzuwandeln, wobei die Umwandlungseinheit (37; 47) sich außerhalb des lebenden Körpers befindet.

3. Strahlenüberwachungsvorrichtung nach Anspruch 1 oder 2, wobei das Identifizierungsmittel ein ID-Tag umfasst, das in einem mit der Umwandlungseinheit (37; 47) verbundenen Speicher gespeichert ist.

4. Strahlenüberwachungsvorrichtung nach Anspruch 3, wobei die Vorrichtung ferner Mittel (37; 47) umfasst, um einen Wert entsprechend der akkumulierten verabreichten Dosis zu berechnen und den berechneten Wert in dem Speicher zu speichern.

5. Strahlenüberwachungsvorrichtung nach einem der Ansprüche 1 bis 4, wobei die entnahmefähige Einheit (44) eine kombinierte Dosis- und Identifizierungseinheit ist.

6. Strahlenüberwachungsvorrichtung nach einem der Ansprüche 1 bis 4, wobei die Führung eine mechanische Führung (32) ist, die zur Vereinigung mit dem Führungselement (36) konfiguriert ist, wenn die entnahmefähige Einheit (34) in den Katheter (31) eingeführt wird, um zu gewährleisten, dass die entnahmefähige Einheit (34) während der Strahlenbehandlung in einer korrekten Position gehalten wird.

7. Strahlenüberwachungsvorrichtung nach Anspruch 6, wobei die entnahmefähige Einheit (34) eine kombinierte Dosis- und Positionierungseinheit ist.

8. Strahlenüberwachungsvorrichtung nach einem der Ansprüche 1 bis 5, wobei das Führungselement ein Steckverbinder (46) ist und die Führung eine elektrische Führung (42) ist, die zur Verbindung mit dem Steckverbinder (46) konfiguriert ist, wenn die entnahmefähige Einheit (44) in den Katheter (41) eingeführt wird, um zu gewährleisten, dass die entnahmefähige Einheit (44) während der Strahlenbehandlung in einer korrekten Position gehalten wird.

9. Strahlenüberwachungsvorrichtung nach Anspruch 8, wobei die Markierung eine elektrische Markierung (43) ist, die zur Verbindung mit der entnahmefähigen Einheit (44) über den Steckverbinder (42) und die elektrische Führung (46) konfiguriert ist.

10. Strahlenüberwachungsvorrichtung nach einem der Ansprüche 1 bis 9, wobei jedes interne Element ein internes Antennenelement ist, das zur Kommunikation unter Verwendung von elektromagnetischen Signalen mit mindestens einem extern angeordneten Antennenelement (75) außerhalb des lebenden Körpers angeordnet ist, um die Position der Vorrichtung relativ zu dem mindestens einen extern angeordneten Antennenelement (75) zu verfolgen.

11. Strahlenüberwachungsvorrichtung nach Anspruch 10, wobei jedes interne Antennenelement konfiguriert ist, um unter Verwendung eines elektromagnetischen Signals zu kommunizieren, das zur Ausbreitung mit einer Wellenlänge in dem lebenden Körper adaptiert ist, so dass eine Phasendifferenz des elektromagnetischen Signals detektierbar ist, um Positionsänderungen jedes internen Antennenelements relativ zu jedem extern angeordneten Antennenelement zu verfolgen.

12. Vorrichtung nach einem der Ansprüche 10 und 11, wobei jedes interne Antennenelement konfiguriert ist, um unter Verwendung eines elektromagnetischen Signals zu kommunizieren, das zur Ausbreitung mit einer Wellenlänge in dem Körper adaptiert ist, so dass eine Amplitudendifferenz des elektromagnetischen Signals detektierbar ist, um Positionsänderungen jedes internen Antennenelements relativ zu jedem extern angeordneten Antennenelement zu verfolgen.

13. Strahlenüberwachungsvorrichtung nach einem der Ansprüche 10 bis 12, wobei das mindestens eine interne Antennenelement ein Sender (Tₓ; 72) ist, der angeordnet ist, um die elektromagnetischen Signale zu emittieren, die in mindestens drei Positionen (78) von dem mindestens einen extern angeordneten Antennenelement (75) detektierbar sind.

14. Strahlenüberwachungsvorrichtung nach einem der Ansprüche 1 bis 11, wobei die Strahlenüberwachungsvorrichtung ein Implantat (30; 40; 79) ist und mindestens ein Teil des Implantats in dem Zielbereich positioniert ist, und wobei das Implantat konfiguriert ist, um mit einer extern angeordneten Steuereinheit (71) verbunden zu werden.

15. System, das die Strahlenüberwachungsvorrichtung (30; 40; 79) nach einem der Ansprüche 1 bis 14 umfasst und wobei die Strahlenquelle (76) zur Anordnung außerhalb des lebenden Körpers (74) konfiguriert ist, wobei die Strahlenüberwachungsvorrichtung (79) zur Einbringung in einen Behandlungsbereich (73) im Inneren des lebenden Körpers konfiguriert ist.

## Revendications

1. Dispositif de contrôle de rayonnement (30; 40 ; 79), pouvant se fixer par rapport à une zone cible (73) à l'intérieur d'un corps vivant (74), dans lequel ledit dispositif comprend au moins un élément interne (Tₓ ; 72) pour suivre les variations d'une position du dispositif par rapport à une source de rayonnement (76) agencée en dehors de ladite zone cible (73) pendant la radiothérapie, ledit dispositif comprenant :
- un cathéter (31 ; 41) muni d'un guide (32 ; 42) et d'un marqueur (33 ; 43), et
- une unité rétractable (34 ; 44) munie d'un élément de guidage (36 ; 46) et d'un dispositif de mesure de dose, configuré pour détecter une dose administrée dans la zone cible à partir de la source de rayonnement lorsque l'unité rétractable (34 ; 44) est positionnée à l'intérieur du cathéter (31 ; 41) et le guide (32 ; 42) s'accouple avec l'élément de guidage (36 ; 46) dans le cathéter (31 ; 41), **caractérisé en ce que** ledit dispositif de contrôle de rayonnement comprend en outre des moyens d'identification (43) pour identifier le corps vivant afin d'assurer que la dose administrée à partir de la source de rayonnement soit rayonnée dans un corps vivant correct.

2. Dispositif de contrôle de rayonnement selon la revendication 1, dans lequel ledit dispositif de mesure de dose comprend au moins un capteur de dose (35 ; 45) agencé à proximité de l'élément interne (Tₓ ; 72), une unité de conversion (37 ; 47) pour convertir le signal provenant du capteur de dose (35 ; 45) en une quantité de dose livrée à la zone cible, ladite unité de conversion (37 ; 47) se trouvant en dehors du corps vivant.

3. Dispositif de contrôle de rayonnement selon la revendication 1 ou 2, dans lequel lesdits moyens d'identification comprennent une balise d'identification stockée dans une mémoire connectée à ladite unité de conversion (37 ; 47).

4. Dispositif de contrôle de rayonnement selon la revendication 3, dans lequel ledit dispositif comprend en outre des moyens (37 ; 47) pour calculer une valeur égale à la dose administrée cumulée et sauvegarder la valeur calculée dans la mémoire.

5. Dispositif de contrôle de rayonnement selon l'une quelconque des revendications 1 à 4, dans lequel ladite unité rétractable (44) est une unité combinée de dosage et d'identification.

6. Dispositif de contrôle de rayonnement selon l'une quelconque des revendications 1 à 4, dans lequel ledit guide est un guide mécanique (32) configuré pour s'accoupler avec ledit élément de guidage (36) lorsque ladite unité rétractable (34) est insérée dans ledit cathéter (31) afin d'assurer que l'unité rétractable (34) soit maintenue dans une position correcte pendant le traitement par rayonnement.

7. Dispositif de contrôle de rayonnement selon la revendication 6, dans lequel ladite unité rétractable (34) est une unité combinée de dosage et de positionnement.

8. Dispositif de contrôle de rayonnement selon l'une quelconque des revendications 1 à 5, dans lequel ledit élément de guidage est un connecteur (46) et ledit guide est un guide électrique (42) configuré pour se connecter au connecteur (46) lorsque ladite unité rétractable (44) est insérée dans ledit cathéter (41) pour assurer que l'unité rétractable (44) soit maintenue dans une position correcte pendant le traitement par rayonnement.

9. Dispositif de contrôle de rayonnement selon la revendication 8, dans lequel le marqueur est un marqueur électrique (43) configuré pour se connecter à l'unité rétractable (44) par ledit connecteur (42) et ledit guide électrique (46).

10. Dispositif de contrôle de rayonnement selon l'une quelconque des revendications 1 à 9, dans lequel chaque élément interne est un élément d'antenne interne, agencé pour communiquer en utilisant des signaux électromagnétiques avec au moins un élément d'antenne (75) agencé en externe, en dehors du corps vivant, pour suivre la position du dispositif par rapport audit au moins un élément d'antenne (75) agencé en externe.

11. Dispositif de contrôle de rayonnement selon la revendication 10, dans lequel chaque élément d'antenne interne est configuré pour communiquer en utilisant un signal électromagnétique qui est adapté pour se propager avec une longueur d'onde dans ledit corps vivant de sorte qu'une différence de phase dudit signal électromagnétique soit décelable pour suivre les variations de la position de chaque élément d'antenne interne par rapport à chaque élément d'antenne agencé en externe.

12. Dispositif selon l'une quelconque des revendications 10 à 11, dans lequel chaque élément d'antenne interne est configuré pour communiquer en utilisant un signal électromagnétique qui est adapté pour se propager avec une longueur d'onde dans ledit corps de sorte qu'une différence d'amplitude dudit signal électromagnétique soit décelable pour suivre les variations de la position de chaque élément d'antenne interne par rapport à chaque élément d'antenne agencé en externe.

13. Dispositif de contrôle de rayonnement selon l'une quelconque des revendications 10 à 12, dans lequel ledit un élément d'antenne interne est un émetteur (Tₓ ; 72) agencé pour émettre les signaux électromagnétiques, qui est décelable dans au moins trois positions (78) par ledit au moins un élément d'antenne (75) agencé en externe.

14. Dispositif de contrôle de rayonnement selon l'une quelconque des revendications 1 à 11, dans lequel ledit dispositif de contrôle de rayonnement est un implant (30 ; 40 ; 79) et au moins une partie de l'implant est positionnée dans la zone cible, et ledit implant est configuré pour se connecter à une unité de commande (71) agencée en externe.

15. Système, comprenant le dispositif de contrôle de rayonnement (30 ; 40 ; 79) selon l'une quelconque des revendications 1 à 14 et ladite source de rayonnement (76) configurée pour être agencée en dehors dudit corps vivant (74), dans lequel ledit dispositif de contrôle de rayonnement (79) est configuré pour être introduit dans une zone de traitement (73) à l'intérieur dudit corps vivant.
